# EUROPEAN PATENT APPLICATION

(11) **EP 3 412 203 A1**
(43) Date of publication of application: **12.12.2018**
(21) Application number: 17174522.7
(22) Date of filing: 06.06.2017
(51) Int. Cl.: A61B 5/0402, A61B 5/0408, A61B 5/0478, A61B 5/0492

(54) **DEVICE FOR MEASURING BIOSIGNALS OF A CREATURE AND CORRESPONDING METHOD**

(71) Applicant: Fraunhofer-Gesellschaft zur Förderung der angewandten Forschung e.V., 80686 München (DE)
(72) Inventor: PFEIFFER, Norman, 96050 Bamberg (DE); HOFMANN, Christian, 90425 Nürnberg (DE)
(74) Representative: Zimmermann, Tankred Klaus

(57) **Abstract**

The invention refers to a measuring device (1) for measuring biosignals of a creature comprising a measurement electrode (2), a stimulation electrode (3) and a control unit (4). The control unit (4) stimulates by means of the stimulation electrode (3) nerve fibers of the body (100) of the creature at a positioning location (P) of the measurement electrode (2), such that sweat is produced at the positioning location (P). The invention also refers to a corresponding method.

## Description

### Specification

The present invention relates to a measuring device for measuring biosignals of a creature. Further, the invention relates to a method for measuring biosignals.

In daily clinical practice, gel electrodes have become widely accepted for detecting biosignals (such as electrocardiography ECG, electromyography EMG, electroencephalography EEG), not least due to their low electrode-skin impedance. Clinical applications of gel electrodes are frequently short-term applications, such as detecting a resting ECG. Disadvantages of gel electrodes are their single-use nature, the low integration ability, e.g., into textile carriers, and possible skin irritations during long-term applications.

Dry electrodes, however, do not show these disadvantages, which is why they are used, among others, in wearables for vital data detection or in prostheses as human-machine interface. Typical materials of dry electrodes are conductive textiles (e.g., fibers evaporated with silver, structures with woven-in stainless steel or silver wire), silicons or polymers. Due to their dry characteristics, very high electrode-skin impedances exist which results in a reduced signal quality in comparison with gel electrodes. In particular directly after applying the electrodes, the electrode-skin impedance is extremely high, since the lack of sweat on the skin surface cannot lower the transition impedance. Due to the lack of electrolytes between skin and electrodes, the measurement is also very susceptible to artifacts due to electrode movements.

For improving the signal quality, two procedures are applied:
By wetting the electrodes in advance, a transition layer of water is introduced between electrode and skin, which can significantly reduce the impedance (electrolytic fluid).

However, this method has the following disadvantages:
- Depending on the electrode material (especially concerning air permeability), the moisture evaporates during longer applications, and thus the signal quality is reduced during a measurement.
- Wetting the electrodes is often perceived as unpleasant by the users.
- It causes a "laborious" application of the measurement system by the additional step of moisturing.
- It is a possible source of errors since the user could moisten the electrode insufficiently or too much.

A different procedure is "waiting for sweat" for the electrodes. Here, the electrodes are positioned on the skin in a dry state and it is waited until a good signal quality is obtained due to the sweat that has been formed under the electrode.

Disadvantages of this method are:
- There can be a long waiting time until the functionality of the system is given, e.g. up to more than 10 minutes.
- In air-permeable electrodes, the sweat evaporates, possibly causing that the electrode-skin impedance constantly remains high.
- The signal quality depends on environmental factors since room humidity and room temperature stimulate perspiration to a different extent.

Apart from these procedures, further systems are known which allow and improve the application of dry electrodes:
The US 8,88,196 B2 describes a textile chest harness fixing integrated defibrillator electrodes on the back and chest of a patient. If the system has to deliver a defibrillator shock in a case of emergency, low skin-electrode transition impedance is required, which is why an electrode gel is automatically provided to the defibrillator electrodes in advance.

Disadvantages of this method are:
- Gel capsules have to be refilled or replaced after usage.
- The system is designed for few and short applications (i.e. the treatment of shocks). Hence, long and frequent applications would quickly use up the existing gel reservoir.
- It might be difficult to integrate the gel chambers in textile carriers for different applications.

The document EP 2 582 290 B1 describes a sensor contact unit that keeps electrodes moist via textiles and a fluid reservoir slowly dispensing the fluid to the electrodes.

Disadvantages of this method are:
- The fluid has to be refilled frequently.
- It might be difficult to integrate the system in textiles or e.g. a prosthesis stem.
- A miniaturization of the sensors is difficult.
- The design might possibly have an impact on the washability.

The document WO 2015/107339 A1 describes a system consisting of electrodes for biosignal detection and mechanical sensors. The mechanical sensors allow detection of electrode shifts since, in particular, dry electrodes are extremely susceptible to artifacts. The knowledge on the mechanical shift relative to the skin allows plausibility check of the signals and/or artifact minimization in the biosignal.

The object of the invention is to provide a measuring device and a corresponding method for measuring biosignals overcoming the drawbacks of the state of art.

The object is achieved by a measuring device for measuring biosignals of a creature. The creature is either a human being or an animal.

The measuring device comprises at least one measurement electrode for determining biosignals of a body of the creature. The biosignal is for example an electric voltage to be measured e.g. for an electrocardiogram, an electromyogram, an electroencephalogram et cetera. The measuring device further comprises at least one stimulation electrode for stimulating nerve fibers of the body in order to effect a perspiration of the body. In one embodiment, the measurement electrode and the stimulation electrode are the same electrode serving as measurement electrode as well as stimulation electrode. Additionally, the measuring device comprises a control unit being coupled with the at least one stimulation electrode. The control unit is configured to stimulate by means of the at least one stimulation electrode nerve fibers of the body at a positioning location of the at least one measurement electrode, such that sweat is produced at the positioning location of the at least one measurement electrode. The sweat serves as an electrolyte between the at least one measurement electrode and the body.

The measuring device comprises at least a measurement electrode and a stimulation electrode. In an embodiment, both functionalities - measurement and stimulation - are realized by a single electrode. A control unit stimulates by means of the stimulation electrode nerve fibers of the body at the positioning location at which the measurement electrode is located. This sweat, thus, reduces the skin-electrode impedance and improves the measurement of the biosignals. In an embodiment, the stimulation electrode serves as a reference electrode for the measurement electrode. In a different embodiment, the stimulation electrode additionally serves as a measurement electrode or the measurement electrode serves as the stimulation electrode.

Hence, in an embodiment, the measurement device allows an electric stimulation of perspiration for influencing the skin-electrode impedance.

The measuring device automatically stimulates perspiration at the at least one measurement electrode positioning location in order to obtain better signal quality and stability by using e.g. dry electrodes for biosignal detection. In an embodiment, perspiration is stimulated at the positioning locations of two measuring electrodes. By this measuring device, the necessity of electrode moistening is omitted and the waiting period for acceptable signal quality in dry applications are reduced. Further, a constant maintenance of the humid environment at the electrode-skin contact area is ensured. Here, the sweat is used as natural electrolyte between electrode and skin of the body. The measuring device allows in an embodiment the measurement of electric potential differences due to electrophysiological signals.

The stimulation electrode triggers the axon reflex perspiration by electric pulses. In axon reflex perspiration, efferent nerve fibers are stimulated, which results in a release of acetylcholine, which again binds to the acetylcholine receptors of the sweat glands and hence results in a sweat response. An additional effect might be a reduction of the impedance between electrode and skin due to increased circulation in the tissue, excited by the electric stimulation.

For the sole purpose of collecting and measuring sweat, document WO 2015/058055 A1 describes electrodes for stimulation of perspiration. Part of the system described in the document is the stimulation of perspiration by applying activating substances, such as pilocarpine or methacholine on the skin surface. An electrical stimulation of sweat is also described in the publication "Diabetes: Sweat Response and Heart Rate Variability During Electrical Stimulation in Controls and People With Diabetes", The Journal of Applied Research, Vol. 8, No. 1, 48 - 54 by Susan Rand, Jerrold S. Petrofsky and Grenith Zimmermann.

According to an embodiment, the control unit is coupled further with the at least one measurement electrode.

In an embodiment, the measuring device is configured for enabling for example electrocardiography, electromyography or electroencephalography of the body. The measurement electrodes are accordingly configured to perform the necessary measurements.

According to an embodiment, the measuring device comprises at least two measurement electrodes.

According to an embodiment, the measuring device comprises at least two stimulation electrodes. Further, the control unit is configured to stimulate by means of the at least two stimulation electrodes the nerve fibers of the body at the positioning location of the at least one measurement electrode. In an embodiment, the at least two stimulation electrodes serve as an electrode to which an electric signal is supplied by the control unit and as a counter electrode. The usage of several stimulation electrodes around a measurement electrode or around various measurement electrodes allows in an embodiment generating a homogeneous distribution of an electric field caused by the stimulation electrodes.

In an alternative embodiment, the measuring device comprises just one single stimulation electrode.

In an embodiment, the at least two stimulation electrodes are arranged around the at least one measurement electrode.

In an embodiment, especially for a multichannel measurement, the perspiration stimulation is performed by at least two stimulation electrodes for several measurement electrodes. This is e.g. the case when the stimulation electrodes surround a measurement electrode array (e.g., in high density EMG) formed by at least two measurement electrodes. In a different embodiment, stimulation takes place between each individual measurement electrode and an associated stimulation electrode.

According to an embodiment, the at least one measurement electrode is configured to serve both as a measurement electrode and as a stimulation electrode. Further, the control unit is configured to supply the at least one measurement electrode with signals in order to stimulate the nerve fibers of the body, such that the measurement electrode acts as the stimulating electrode. Hence, at least one measurement electrode also serves as a stimulation electrode. Accordingly, it might be called measurement and stimulation electrode. Due to this design, the control unit receives in an embodiment from this measurement and stimulation electrode signals and supplies it with signals for stimulating perspiration.

In a further embodiment, an additional stimulation electrode or another measurement electrode is used as counter-electrode for the measurement and stimulation electrode.

In an embodiment, the measuring device comprises a sensor. The sensor is configured to provide a sensor signal being dependent on the sweat produced at the positioning location of the at least one measurement electrode. The sensor is, for example, a humidity sensor, a sweat rate sensor or performs an impedance measurement. The sensor is used as input for a control loop affecting the stimulation signal.

According to an embodiment, the control unit is configured to determine the biosignals by means of the at least one measurement electrode and to stimulate the nerve fibers of the body by means of the at least one stimulating electrode based on the sensor signal of the sensor. In this embodiment, the stimulation of perspiration depends on the sensor signals provided by the aforementioned sensor. As the sensor signals are dependent on sweat, a kind of feedback control is installed using the sensor and the control unit. Hence, the controlling of stimulation and measurement is done depending on the results of the measurements of the sensor. Hence, a control loop (controlled e.g. by impedance measurement, humidity measurement, signal quality, measuring the sweat rate, etc.) ensures a permanent and constant humidity film between skin and electrode by switching the stimulation on and off and/or by modifying the stimulation signals.

In an embodiment, the at least one measurement electrode and/or the at least one stimulating electrode serve/serves as the sensor. In this embodiment, at least one electrode (measurement electrode or stimulating electrode) or both electrodes serves/serve as the sensor and provide the sensor signals being dependent on the perspiration caused by the stimulation electrode.

In an alternative or additional embodiment, the control unit is configured to determine the biosignals by means of the at least one measurement electrode and to stimulate the nerve fibers of the body by means of the at least one stimulating electrode based on a measurement of the at least one measurement electrode. Here, the measurement and the stimulation of perspiration are controlled by the control unit using the result of a measurement. This refers for example to the signal-to-noise-ratio of a measurement signal or to a comparison of the - current - measurement with foregoing measurements.

In an embodiment, the control unit is configured to supply the at least one stimulation electrode with electric signals - for example in the form of pulses - for stimulating the nerve fibers.

According to an embodiment, the control unit is configured to set a frequency and/or an amplitude and/or a signal form and/or a duty cycle and/or a phase of the electric signals.

If in an embodiment the stimulation is performed at the same time as the biosignal measurement, the current amplitudes of the stimulation signals are preferably adjusted such that the input amplifier of the measurement electrode is not driven into saturation.

In a different embodiment, for interference minimization of the measurement by the stimulation, adaptations are made as regards to e.g. wave form, signal amplitude or signal frequency.

In an embodiment, the control unit is configured to determine the biosignals by means of the at least one measurement electrode and to stimulate the nerve fibers of the body by means of the at least one stimulating electrode at the same time.

In an embodiment, stimulation takes place continuously between the sample processes of an analog-digital-converter connected with the measurement electrode.

Triggering axon reflex perspiration by permanent stimulation prior to the beginning of a measurement is performed in an embodiment in order to quickly obtain the desired humidity between electrode and skin.

In an embodiment, the control unit is configured to stimulate the nerve fibers of the body by means of the at least one stimulating electrode during a predetermined stimulation time interval. The control unit is configured to determine the biosignals by means of the at least one measurement electrode during a predetermined measuring time interval following the stimulation time interval. Here, a temporal sequential sequence is used for stimulation and measurement in order to prevent an interference of the measurement. The stimulation time interval and the measuring time interval do not overlap in this embodiment.

According to an embodiment, the control unit is configured to determine the biosignals during the predetermined measuring time interval immediately following the stimulation time interval. Hence, there is no pause between stimulation and measurement. Thus, in this embodiment, stimulation is followed by measuring being followed by stimulation and so on.

In a different embodiment, an external trigger signal is used for initiating and/or stopping the stimulation. The external trigger signal indicates, for example, that biosignals are not required for a moment or that due to specific conditions, e.g. an activity of the creature, it is momentarily not feasible to perform a reliable measurement. Such context information is obtained in an embodiment via sensors, e.g. an acceleration sensor.

In an embodiment, the at least one measurement electrode is a dry electrode.

In an embodiment, further physiological phenomena are used for improving the electrode-skin contact. In an embodiment, piloerections (goosebumps) are generated for an improvement of the electrode-skin contact. In a different embodiment, improvement of the signal quality is achieved as the sweat ducts of the skin are filled with sweat and hence improved electric conductivity through the epidermis is made possible. Also a higher blood flow can reduced the impedance.

The length of stimulating perspiration is set in an embodiment by the control unit based on measured values, as e.g. humidity, impedance or signal quality.

In an embodiment, perspiration-stimulating substances are administered to the body, which are moved to specific positions, for example by iontophoresis.

In an embodiment, the measurement electrodes are heated measurement electrodes and the control unit supply the heated measurement electrodes with heating signals such that the heated measurement electrodes heat the body at the positioning location. This leads to a higher blood flow and an increased sweating rate.

Some advantages are as follows:
By continuous regulation of the skin-electrode impedance by axon reflex perspiration, biosignals can be detected in a noise free and stable manner. Additionally, improved adhesion of the electrode on the skin can be obtained by the sweat film, whereby movement artifacts by mechanical influences are minimized. These characteristics allow a practical application for detecting physiological data. For example, EMG detection for controlling myoelectric prostheses requires high reliability for preventing erroneous functions and to obtain high acceptance by the patient. Further, chest straps and smart textiles implemented for detecting biosignals, such as ECGs, can be provided with additional electrodes for perspiration stimulation. Thus, minimization of artifact susceptibility and improvement of the signal-to-noise ratio is obtained.

The advantage of the measurement device is that the skin-electrode impedance is actively regulated, the same can hence be kept constant and high signal quality is obtained. In contrary to other known systems, no additional electrolyte and no regular refilling is necessary. Since the stimulation electrodes can be integrated into a carrier similar to the measurement electrodes, significantly higher integration ability is given compared to systems with additional electrolyte.

Possible applications are:
- Wearables for biosignal detection in the lifestyle sector,
- medical measurement systems for long-term measurements,
- measurement systems for biosignal detection for sports science,
- man-machine interfaces based on biosignals (e.g., EEG, EMG),
- myoelectric prostheses,
- electrostimulation (EMS) training or
- stimulation current and electrotherapy in the field of stress management, bio feedback, relaxation.

The object is also achieved by a method for measuring biosignals of a creature.

The method comprises at least the following steps:
- Stimulating nerve fibers of a body of the creature by means of at least one stimulation electrode in order to effect a perspiration of the body at least at a positioning location of at least one measuring electrode, such that sweat is produced at the positioning location of the at least one measurement electrode.
- Determining biosignals of the body by means of the at least one measurement electrode.

The above discussed embodiments and features of the measurement device can also be realized via the method and vice versa.

The invention will be explained in the following with regard to the accompanying drawings and the embodiments depicted in the accompanying drawings, in which:
- Fig. 1: illustrates a measurement using the measuring device,
- Fig. 2: shows an embodiment of the measuring device,
- Fig. 3: shows a different embodiment of the measuring device and
- Fig. 4: shows a further embodiment of the measuring device.

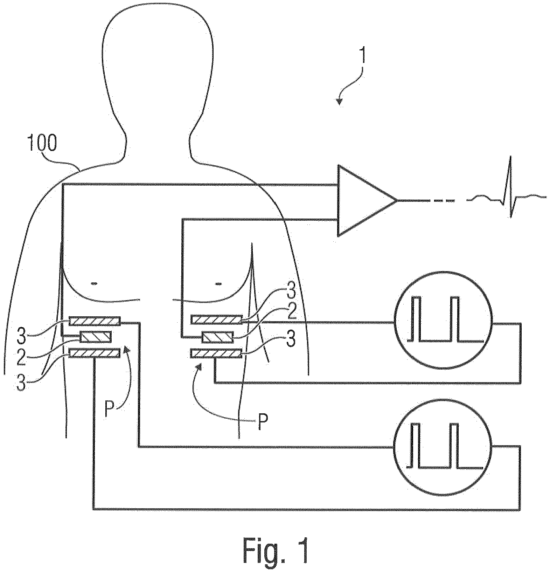

Fig. 1 illustrates a measurement of biosignals of a body 100 of a creature - here a human being - using the measuring device 1.

In the shown embodiment, the measuring device 1 comprises six electrodes: two measurement electrodes 2 and four stimulation electrodes 3 where two stimulation electrodes 3 are arranged around each measurement electrode 2.

The measurement electrodes 2 for a single-channel ECG measurement are positioned at two positioning locations P and allow in the shown embodiment the measurement of an electrocardiogram of the body 100. For an improved contact between the measurement electrodes 2 and the body 100, the stimulation electrodes 3 are used.

The stimulation electrodes 3 stimulate the efferent nerve fibers under the respective measurement electrode 2 by electric impulses, whereby acetylcholine is released which again stimulates the activity of sweat glands. This results in an increased local sweat rate, especially at the positioning locations P. Additionally, circulation of the affected tissue is increased. The sweat serves as natural electrolyte between measuring electrode 2 and skin of the body 100 and results in a more stable measurement of biosignals due to low skin-electrode impedance.

Hence, in the shown embodiment two stimulation electrodes 3 are used for stimulating perspiration at the positioning location P of each measurement electrode 2. In a different - and not shown - embodiment, the stimulation electrodes 3 are located around an array of measurement electrodes 2.

For the stimulation, the stimulation electrodes 3 are supplied with electric signals, here electric pulses where the signal form, the amplitude and/or the frequency of the pulses are set according to the requirements of the measurement.

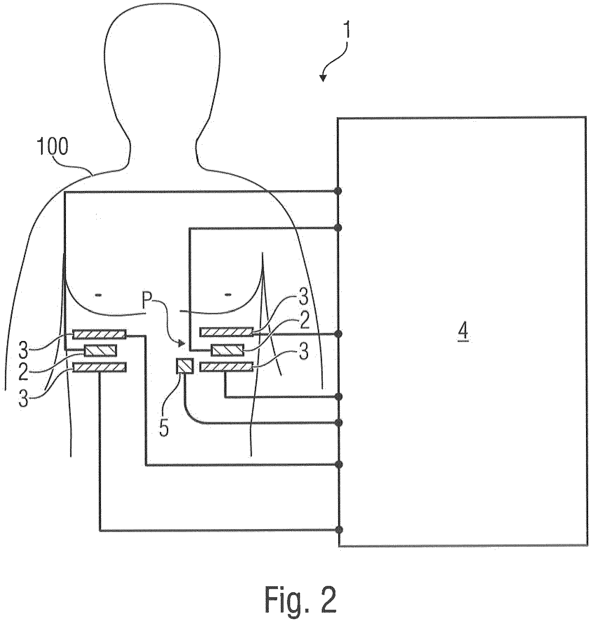

The measuring device 1 of Fig. 2 shows the control unit 4 being connected in this embodiment with two measurement electrodes 2 and two stimulation electrodes 3 for each measurement electrode 2. Additionally, a sensor 5 is located close to one measurement electrode 2. This sensor 5 provides a sensor signal that is dependent on the sweat in its vicinity and, thus, at the positioning location P of the measurement electrode 2. The sensor signal allows the control unit 4 to set the parameters of the signals supplied to the stimulation electrodes 3 in order to achieve desired measurement conditions for the measurement electrodes 2.

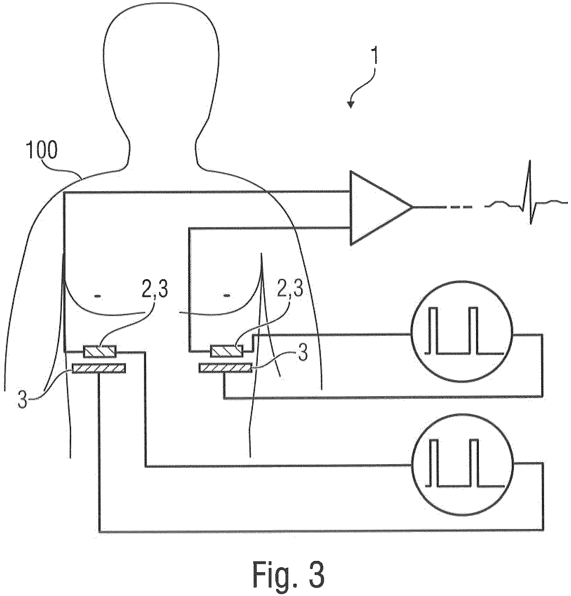

The embodiment shown in Fig. 3 differs from the embodiment of Fig. 1 as two measurement and stimulation electrodes 2, 3 are given which serve as measurement electrodes 2 as well as stimulation electrodes 3. Hence, all-in-all four electrodes are used. In the shown embodiment, for each measurement and stimulation electrode 2, 3 a stimulation electrode 3 is located close by. For the stimulation of the perspiration, electric pulses are supplied to the measurement and stimulation electrode 2, 3 and to the respective stimulation electrode 3. For the measurement of the ECG, signals are tapped from the measurement and stimulation electrodes 2, 3.

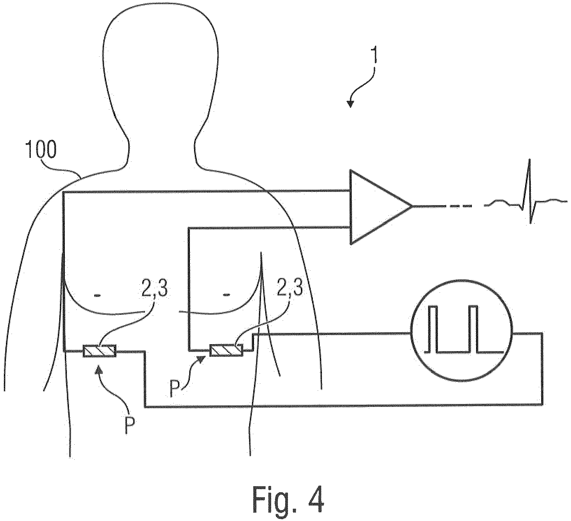

The measurement device 1 shown in Fig. 4 comprises just two electrodes which serve both as measurement electrode 2 and stimulation electrode 3 and which are located at the two positioning locations P. For the stimulation purpose, both measurement and stimulation electrodes 2, 3 are supplied with the stimulation signal. For the measurement, both measurement and stimulation electrodes 2, 3 are used for measuring the biosignals of the body 100.

Although some aspects have been described in the context of an apparatus, it is clear that these aspects also represent a description of the corresponding method. Analogously, aspects described in the context of a method step also represent a description of a corresponding block or item or feature of a corresponding apparatus.

## Claims

1. A measuring device (1) for measuring biosignals of a creature, comprising:
at least one measurement electrode (2) for determining biosignals of a body (100) of the creature,
at least one stimulation electrode (3) for stimulating nerve fibers of the body (100) in order to effect a perspiration of the body (100), and
a control unit (4) being coupled with the at least one stimulation electrode (3),
wherein the control unit (4) is configured to stimulate by means of the at least one stimulation electrode (3) nerve fibers of the body (100) at a positioning location (P) of the at least one measurement electrode (2), such that sweat is produced at the positioning location (P) of the at least one measurement electrode (2).

2. The measuring device (1) of claim 1,
wherein the control unit (4) being coupled further with the at least one measurement electrode (2).

3. The measuring device (1) of claim 1 or 2,
wherein the measuring device (1) comprises at least two measurement electrodes (2).

4. The measuring device (1) of any of claims 1 to 3,
wherein the measuring device (1) comprises at least two stimulation electrodes (3), and
wherein the control unit (4) is configured to stimulate by means of the at least two stimulation electrodes (3) the nerve fibers of the body (100) at the positioning location of the at least one measurement electrode (2).

5. The measuring device (1) of any of claims 1 to 4,
wherein the at least one measurement electrode (2) is configured to serve both as a measurement electrode (2) and as a stimulation electrode (3), and wherein the control unit (4) is configured to supply the at least one measurement electrode (2) with signals in order to stimulate the nerve fibers of the body (100), such that the measurement electrode (2) acts as the stimulating electrode (3).

6. The measuring device (1) of any of claims 1 to 5,
wherein the measuring device (1) comprises a sensor (5), and
wherein the sensor (5) is configured to provide a sensor signal being dependent on the sweat produced at the positioning location of the at least one measurement electrode (2).

7. The measuring device (1) of claim 6,
wherein the control unit (4) is configured to determine the biosignals by means of the at least one measurement electrode (2) and to stimulate the nerve fibers of the body (100) by means of the at least one stimulating electrode (3) based on the sensor signal of the sensor (5).

8. The measuring device (1) of claim 6 or 7,
wherein the at least one measurement electrode (2) and/or the at least one stimulating electrode (3) serve/serves as the sensor (5).

9. The measuring device (1) of any of claims 1 to 8,
wherein the control unit (4) is configured to determine the biosignals by means of the at least one measurement electrode (2) and to stimulate the nerve fibers of the body (100) by means of the at least one stimulating electrode (3) based on a measurement of the at least one measurement electrode (2).

10. The measuring device (1) of any of claims 1 to 9,
wherein the control unit (4) is configured to supply the at least one stimulation electrode (3) electric signals for stimulating the nerve fibers.

11. The measuring device (1) of claim 10,
wherein the control unit (4) is configured to set a frequency and/or an amplitude and/or a signal form and/or a duty cycle and/or a phase of the electric signals.

12. The measuring device (1) of any of claims 1 to 11,
wherein the control unit (4) is configured to determine the biosignals by means of the at least one measurement electrode (2) and to stimulate the nerve fibers of the body (100) by means of the at least one stimulating electrode (3) at the same time.

13. The measuring device (1) of any of claims 1 to 11,
wherein the control unit (4) is configured to stimulate the nerve fibers of the body (100) by means of the at least one stimulating electrode (3) during a predetermined stimulation time interval, and
wherein the control unit (4) is configured to determine the biosignals by means of the at least one measurement electrode (2) during a predetermined measuring time interval following the stimulation time interval.

14. The measuring device (1) of any of claims 1 to 13,
wherein the at least one measurement electrode (2) is a dry electrode.

15. A method for measuring biosignals of a creature,
comprising:
stimulating nerve fibers of a body (100) of the creature by means of at least one stimulation electrode (3) in order to effect a perspiration of the body (100) at least at a positioning location (P) of at least one measuring electrode (2), such that sweat is produced at the positioning location (P) of the at least one measurement electrode (2), and
determining biosignals of the body (100) by means of the at least one measurement electrode (2).
